(19)

Europäisches
Patentamt
European
Patent Office
Office européen
des brevets

(11) **EP 4 239 334 A1**

(12) **EUROPEAN PATENT APPLICATION**

(43) Date of publication:
**06.09.2023 Bulletin 2023/36**

(21) Application number: **22382186.9**

(22) Date of filing: **02.03.2022**

(51) International Patent Classification (IPC):
**G01N 33/50** *(2006.01)*      *C12Q 1/6886* *(2018.01)*
**G01N 33/574** *(2006.01)*

(52) Cooperative Patent Classification (CPC):
**G01N 33/5011; A61P 35/00;** C12Q 1/6886;
G01N 33/57407; G01N 33/57423; G01N 2500/10;
G01N 2800/52; G01N 2800/56

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Consorcio Centro de Investigación Biomédica en
Red
(CIBER)
28029 Madrid (ES)**
• **Fundació Institut Mar d'Investigacions Mèdiques
(IMIM)
08003 Barcelona (ES)**

(72) Inventors:
• **ESPINOSA BLAY, Lluís**
**08003 Barcelona (ES)**
• **BIGAS SALVANS, Anna**
**08003 BARCELONA (ES)**
• **SOLÉ FONT, Laura**
**28029 Madrid (ES)**

(74) Representative: **Hoffmann Eitle**
**Hoffmann Eitle S.L.U.**
**Paseo de la Castellana 140, 3a planta
Edificio LIMA
28046 Madrid (ES)**

Remarks:
The complete document including Reference
Table(s) and the Sequence Listing(s) can be
downloaded from the EPO website

(54) **SEPARATION-OF-FUNCTION I KAPPA B ALPHA MUTANTS**

(57)     The present invention refers to specific IκBα mutants which can be used for deciding or recommending a treatment to a patient suffering from a cancer type characterized by the inactivation of IκBα protein or for the identification of therapeutic targets or biomarkers for the treatment or diagnosis of a cancer type characterized by the inactivation of IκBα protein.

EP 4 239 334 A1

**Description**

**FIELD OF THE INVENTION**

The present invention refers to the medical field. Particularly, the present invention refers to specific IκBα mutants which can be used for deciding or recommending a treatment to a patient suffering from a cancer type characterized by the inactivation of IκBα protein or for the identification of therapeutic targets or biomarkers for the treatment or diagnosis of a cancer type characterized by the inactivation of IκBα protein.

**STATE OF THE ART**

NF-κB pathway is an essential regulator of inflammation, immune response and cellular survival, being IκB family of proteins the primary inhibitors of the pathway both under non-stimulated conditions and following activation. Canonical NF-κB is initiated by activation of the IKK kinase complex that phosphorylates IκBs at specific serine (S) residues (S32 and 36 in the case IκBα) thus inducing their β-TRCP-dependent K48-linked ubiquitination and proteasomal degradation. For years, negative regulation of NF-κB has been considered the only function for IκBα although alternative post-translational modifications of IκBα have also been shown to affect its activity under specific situations. SUMOylation of IκBα has initially been described associated with the non-phosphorylated IκBα forms thus preventing degradation and NF-κB activation. Contrary, IκBα SUMOylation induced by hypoxia facilitates p65 release and NF-κB activation. Other stimuli such as $H_2O_2$, epidermal growth factor or pervanadate induce tyrosine (Y) phosphorylation of IκBα (at Y42) thus leading to NF-κB activation independent of IκBα degradation. Phosphorylation of IκBα at Y42 was found to be required for proper neuron development. Alternative non-degradative phosphorylation of Y289 and Y305 of IκBα promotes NF-κB activation in B cells.

Previous results from our group demonstrated that SUMOylated IκBα is predominantly nuclear and associates with histones in the chromatin to modulate the activity of polycomb repressor complex 2 (PRC2) at a subset of stemness- and developmental-related genes. Moreover, association of IκBα with NF-κB or histone H4 in vitro is mutually exclusive, suggesting that a common domain in IκBα mediates both interactions. Further investigations indicated that developmental defects associated with IκBα deficiency are linked to its PRC2-related function. However, due to the predominant role of IκBα as regulator of NF-κB, the broad spectrum of NF-κB activities and the multiple crosstalk between NF-κB and PRC2, uncovering the functional impact of PRC2-related IκBα activity in specific systems has remained extremely challenging. This is an important issue with clinical implication, since impaired IκBα activity has recurrently been observed in various types of human cancer such as glioblastoma, low grade glioma, non-small cell lung carcinoma, gray zone lymphoma or Hodgkin lymphoma, as well as in different murine models of skin cancer but revealing the actual impact of aberrant NF-κB signaling to tumor initiation or progression in each particular case has remained elusive.

There is an unmet medical need of finding reliable strategies for assessing whether a cancer type characterized by the inactivation of IκBα protein has been originated via activation of NF-κB or, alternatively, via PRC2. The identification of this strategy would have a clear impact in patient treatment management since, once the specific pathway is identified, specific inhibitors directed towards blocking specific pathways could be used. This approach could be also used to stratify patients to be included in clinical trials.

The present invention is indeed focused on solving this problem and, consequently, an innovative strategy for assessing whether a cancer type characterized by the inactivation of IκBα protein has been originated via activation of NF-κB or, alternatively, via PRC2 has been designed, which comprises the use of specific IκBα mutants.

**DESCRIPTION OF THE INVENTION**

**Brief description of the invention**

As explained above, the the present invention refers to specific IκBα (nuclear factor of kappa light polypeptide gene enhancer in B-cells inhibitor, alpha) mutants which are herein used for assessing whether a cancer type characterized by the inactivation of IκBα protein has been originated via activation of NF-κB (nuclear factor kappa-light-chain-enhancer of activated B cells) or, alternatively, via PRC2 (polycomb repressive complex 2). So, the present invention can be used for deciding or recommending a treatment to a patient suffering from a cancer type characterized by the inactivation of IκBα protein or for the identification of therapeutic targets or biomarkers for the treatment or diagnosis of a cancer type characterized by the inactivation of IκBα protein.

IκBα is one member of a family of cellular proteins that function to inhibit the NF-κB transcription factor. IκBα inhibits NF-κB by masking the nuclear localization signals of NF-κB proteins and keeping them sequestered in an inactive state in the cytoplasm. In addition, IκBα blocks the ability of NF-κB transcription factors to bind to DNA, which is required for NF-κB's proper functioning.

The gene encoding the IκBα protein is mutated in some cancer cells. Such mutations inactivate the IκBα protein, thus causing NF-κB to be chronically active in the tumor cells and this activity contributes to the malignant state of these tumor cells.

However, IκBα apart from acting over NF-κB, can be alternatively involved in a different pathway, regulating polycomb repression complex 2 (PRC2).

The inventors of the present invention have identified key residues of the IκBα protein that determine if the function of IκBα is occurring over NF-κB (classical function) or, alternatively, over PRC2. Particularly, the inventors of the present invention have generated IκBα mutants which can be used to discriminate, in each case, the above cited two main functions of IκBα. These mutants are identified in the present report as: IκBα SOF $^{PRC2}$ (specific function over PRC2) and IκBα SOF $^{NF-κB}$ (specific function over NF-κB).

From a translational perspective, the inventors of the present invention propose obtaining cells from patients with tumors associated with loss of IκBα (e.g. Hodgkin's lymphoma, squamous cell carcinoma or glioblastoma) and reconstitute these cells with lentiviral vectors encoding for wild-type IκB, IκBα SOF $^{PRC2}$ or IκBα SOF $^{NF-κB}$. Then, tumor activity of the cells transfected with vectors encoding IκBα SOF PRC2 or IκBα SOF $^{NF-κB}$ would be compared, by *in vitro* and *in vivo* assays, with respect to the original IκBα-deficient cells, thus giving rise to the opportunity of defining which pro-tumorigenic pathway is active in each type of IκBα-deficient tumor, which would lead to a better definition of therapeutic target pathways in each tumor type.

Consequently, it is expected to identify IκBα-deficient tumors whose pro-tumorigenic pathway is associated with NF-κB when the tumor activity is reversed by transfecting the cells with a vector encoding IκBα SOF $^{NF-κB}$. Alternatively, it is expected to identify IκBα-deficient tumors whose pro-tumorigenic pathway is associated with PRC2 when the tumor activity is reversed by transfecting cells with a vector encoding IκBα SOF $^{PRC2}$.

This approach has direct applications in patient treatment management since, once the specific pathway is identified, specific inhibitors directed towards blocking specific pathways could be used. This approach could be also used to stratify patients to be included in clinical trials.

Moreover, a transcriptomic and proteomic study of reconstituted cells with different degrees of tumorigenicity could be carried out, which would allow to define specific alterations/pathways (which may represent new therapeutic targets) as well as biomarkers (or transcriptional signatures) with future clinical utility.

So, the first embodiment of the present invention refers to a IκBα mutant characterized by the **SEQ ID NO: 1** (IκBα SOF $^{NF-κB}$), **SEQ ID NO: 2** (IκBα SOF $^{PRC2}$) or **SEQ ID NO: 3** (IκBα SOF $^{PRC2}$). In other words, the IκBα mutants of the invention comprise or consist of **SEQ ID NO: 1**, **SEQ ID NO: 2** or **SEQ ID NO: 3**.

Particularly, the sequences of the IκBα mutants of the invention are as follows:

Amino acid sequence (IkBa HEE84-86AAA/SOF$^{NF-kB}$) (**SEQ ID NO: 1**)

MFQAAERPQEWAMEGPRDGLKKERLLDDRHDSGLDSMKDEEYEQMVKELQEIRLEPQEVP 60

RGSEPWKQQLTEDGDSFLHLAII**AAA**KALTMEVIRQVKGDLAFLNFQNNLQQTPLHLAVI 120

TNQPEIAEALLGAGCDPELRDFRGNTPLHLACEQGCLASVGVLTQSCTTPHLHSILKATN 180

YNGHTCLHLASIHGYLGIVELLVSLGADVNAQEPCNGRTALHLAVDLQNPDLVSLLLKCG 240

ADVNRVTYQGYSPYQLTWGRPSTRIQQQLGQLTLENLQMLPESEDEESYDTESEFTEFTE 300

DELPYDDCVFGGQRLTL 317

Amino acid sequence (IkBa D75A/SOF$^{PRC2}$) (**SEQ ID NO: 2**)

MFQAAERPQEWAMEGPRDGLKKERLLDDRHDSGLDSMKDEEYEQMVKELQEIRLEPQEVP 60

RGSEPWKQQLTEDG**A**SFLHLAIIHEEKALTMEVIRQVKGDLAFLNFQNNLQQTPLHLAVI 120

TNQPEIAEALLGAGCDPELRDFRGNTPLHLACEQGCLASVGVLTQSCTTPHLHSILKATN 180

YNGHTCLHLASIHGYLGIVELLVSLGADVNAQEPCNGRTALHLAVDLQNPDLVSLLLKCG 240

ADVNRVTYQGYSPYQLTWGRPSTRIQQQLGQLTLENLQMLPESEDEESYDTESEFTEFTE 300

DELPYDDCVFGGQRLTL 317

Amino acid sequence (IkBa N108A/SOF$^{PRC2}$) **(SEQ ID NO: 3)**

MFQAAERPQEWAMEGPRDGLKKERLLDDRHDSGLDSMKDEEYEQMVKELQEIRLEPQEVP 60

RGSEPWKQQLTEDGDSFLHLAIIHEEKALTMEVIRQVKGDLAFLNFQ**A**NLQQTPLHLAVI 120

TNQPEIAEALLGAGCDPELRDFRGNTPLHLACEQGCLASVGVLTQSCTTPHLHSILKATN 180

YNGHTCLHLASIHGYLGIVELLVSLGADVNAQEPCNGRTALHLAVDLQNPDLVSLLLKCG 240

ADVNRVTYQGYSPYQLTWGRPSTRIQQQLGQLTLENLQMLPESEDEESYDTESEFTEFTE 300

DELPYDDCVFGGQRLTL 317

On the other hand, the sequence of the IkBa wild type is as follows:
Amino acid sequence (IkBa wild type) **(SEQ ID NO: 4)**

MFQAAERPQEWAMEGPRDGLKKERLLDDRHDSGLDSMKDEEYEQMVKELQEIRLEPQEVP 60

RGSEPWKQQLTEDGD**D**SFLHLAII**HEE**KALTMEVIRQVKGDLAFLNFQ**N**NLQQTPLHLAVI 120

TNQPEIAEALLGAGCDPELRDFRGNTPLHLACEQGCLASVGVLTQSCTTPHLHSILKATN 180

YNGHTCLHLASIHGYLGIVELLVSLGADVNAQEPCNGRTALHLAVDLQNPDLVSLLLKCG 240

ADVNRVTYQGYSPYQLTWGRPSTRIQQQLGQLTLENLQMLPESEDEESYDTESEFTEFTE 300

DELPYDDCVFGGQRLTL 317

The second embodiment of the present invention refers to genetic vector encoding the IκBα mutants of the invention.

The third embodiment of the present invention refers to an *in vitro* method for deciding or recommending a treatment to a patient suffering cancer, wherein the cancer is characterized by the inactivation of IκBα protein, which comprises:

a. Transfecting tumor cells obtained from the patient with a genetic vector which encodes the mutant IκBα SOF $^{NF-κB}$ of **SEQ ID NO: 1**, or the mutant IκBα SOF $^{PRC2}$ of **SEQ ID NO: 2** or **SEQ ID NO: 3**;
b. Wherein the identification of a reduction or elimination of tumorigenicity once the tumor cells have been transfected with a genetic vector encoding IκBα SOF $^{NF-κB}$ of **SEQ ID NO: 1**, as compared with a pre-established tumorigenicity observed before transfecting the cells, is an indication that the tumor has been originated via activation of NF-κB, and a treatment directed to the inhibition of NF-κB pathway would be recommended; or
c. Wherein the identification of a reduction or elimination of tumorigenicity once the tumor cells have been transfected with a genetic vector encoding the mutant IκBα SOF $^{PRC2}$ of **SEQ ID NO: 2** or **SEQ ID NO: 3**, as compared with a pre-established tumorigenicity observed before transfecting the cells, is an indication that the tumor has been originated via PRC2, and a treatment directed to the inhibition of PRC2 pathway would be recommended.

The fourth embodiment of the present invention refers to an *in vitro* method for the identification of therapeutic targets or biomarkers for the treatment or diagnosis of a cancer type characterized by the inactivation of IκBα protein, which comprises:

a. Performing a transcriptomic or proteomic study of reconstituted cells transfected with a genetic vector encoding the mutant IκBα SOF $^{NF-κB}$ of **SEQ ID NO: 1**, or the mutant IκBα SOF $^{PRC2}$ of **SEQ ID NO: 2** or **SEQ ID NO: 3;**
b. Performing a transcriptomic or proteomic study of the cells before being transfected;
c. Wherein the identification of a deviation in the level of expression of a particular gene or protein, is an indication that the protein or gene can be used as a therapeutic target or biomarker for the treatment or diagnosis of a cancer type characterized by the inactivation of IκBα protein.

The fifth embodiment of the present invention refers to NF-κB inhibitors for use in the treatment of patients suffering from a cancer type characterized by the inactivation of IκBα protein, which comprises assessing whether the cancer has been actually originated via activation of NF-κB by transfecting patient cells with a genetic vector encoding the mutant IκBα SOF NF-κB of **SEQ ID NO: 1**. Alternatively, the present invention refers to a method for treating a patients suffering from a cancer type characterized by the inactivation of IκBα protein, which comprises assessing whether the cancer has been actually originated via activation of NF-κB by transfecting patient cells with a genetic vector encoding the mutant IκBα SOF NF-κB of **SEQ ID NO: 1**, and treating the patient with a therapeutically effective amount of NF-κB inhibitors.

The sixth embodiment of the present invention refers to PRC2 inhibitors for use in the treatment of patients suffering from a cancer type characterized by the inactivation of IκBα protein, which comprises assessing whether the cancer has been actually originated via activation of PRC2 by transfecting patient cells with a genetic vector encoding the mutant IκBα SOF PRC2 of **SEQ ID NO: 2** or **SEQ ID NO: 3**. Alternatively, the present invention refers to a method for treating a patients suffering from a cancer type characterized by the inactivation of IκBα protein, which comprises assessing whether the cancer has been actually originated via activation of PRC2 by transfecting patient cells with a genetic vector encoding the mutant IκBα SOF PRC2 of **SEQ ID NO: 2** or **SEQ ID NO: 3**, and treating the patient with therapeutically effective amount PRC2 inhibitors.

In a preferred embodiment, the cancer characterized by the inactivation of IκBα protein is selected from Hodgkin's lymphoma, liver cancer, squamous cell carcinoma or glioblastoma. The inactivation of IκBα protein in several indications have been documented in the prior art and pertains to the common general knowledge. See for instance: [Emmerich F, Meiser M, Hummel M, et al. Overexpression of I kappa B alpha without inhibition of NF-kappaB activity and mutations in the I kappa B alpha gene in Reed-Sternberg cells. Blood. Nov 01 1999;94(9):3129-34], [Cabannes E, Khan G, Aillet F, Jarrett RF, Hay RT. Mutations in the IkBa gene in Hodgkin's disease suggest a tumour suppressor role for IkappaBalpha. Oncogene. May 20 1999;18(20):3063-70. doi:10.1038/sj.onc.1202893], [Jungnickel B, Staratschek-Jox A, Bräuninger A, et al. Clonal deleterious mutations in the IkappaBalpha gene in the malignant cells in Hodgkin's lymphoma. J Exp Med. Jan 17 2000; 191(2):395-402. doi:10.1084/jem.191.2.395], [Zhao Z, Zhong X, Wu T, et al. Identification of a NFKBIA polymorphism associated with lower NFKBIA protein levels and poor survival outcomes in patients with glioblastoma multiforme. Int J Mol Med. Nov 2014;34(5):1233-40. doi:10.3892/ijmm.2014.1932], [BredelM, Scholtens DM, Yadav AK, et al. NFKBIA deletion in glioblastomas. N Engl J Med. Feb 2011 ;364(7):627-37. doi:10.1056/NEJMoa1006312], [He Y, Zhang H, Yin J, et al. IkappaBalpha gene promoter polymorphisms are associated with hepatocarcinogenesis in patients infected with hepatitis B virus genotype C. Carcinogenesis. Nov 2009;30(11):1916-22. doi:10.1093/carcin/bgp226], [Parker KM, Ma MH, Manyak S, et al. Identification of polymorphisms of the IkappaBalpha gene associated with an increased risk of multiple myeloma. Cancer Genet Cytogenet. Aug 2002;137(1):43-8. doi:10.1016/s0165-4608(02)00541-1i or [Mulero MC, Ferres-Marco D, Islam A, et al. Chromatin-bound IκBα regulates a subset of polycomb target genes in differentiation and cancer. Cancer Cell. Aug 2013;24(2):151-66. doi:10.1016/j.ccr.2013.06.003] wherein it is shown that IkBa is lost in squamous cell carcinoma.

In a preferred embodiment of the present invention, the treatment is directed to the inhibition of NF-κB pathway. Treatments directed to the inhibition of NF-κB pathway have been disclosed before and pertains to the common general knowledge. See for instance: [Yu H, Lin L, Zhang Z, Zhang H, Hu H. Targeting NF-κB pathway for the therapy of diseases: mechanism and clinical study. Signal Transduct Target Ther. 09 21 2020;5(1):209. doi:10.1038/s41392-020-00312-6] or [Durand JK, Baldwin AS. Targeting IKK and NF-κB for Therapy. Adv Protein Chem Struct Biol. 2017 2017;107:77-115. doi:10.1016/bs.apcsb.201611.006].

In a preferred embodiment of the present invention, the treatment is directed to the inhibition of PRC2 pathway. Treatments directed to the inhibition of PRC2 pathway have been disclosed before and pertains to the common general knowledge. See for instance: [*Cheng Y, He C, Wang M, et al. Targeting epigenetic regulators for cancer therapy: mechanisms and advances in clinical trials. Signal Transduct Target Ther. 2019 2019;4:62. doi:10.1038/s41392-019-0095-0], [Dawson MA, Kouzarides T. Cancer epigenetics: from mechanism to therapy. Cell. Jul 6 2012;150(1):12-27. doi:10.1016/j.cell.2012.06.013] or [Piunti A, Hashizume R, Morgan MA, et al. Therapeutic targeting of polycomb and BET bromodomain proteins in diffuse intrinsic pontine gliomas. Nat Med. Apr 2017;23(4):493-500. doi:10.1038/nm.4296]*.

For the purpose of the present invention, the following terms are defined:

- The term "comprising" means including, but not limited to, whatever follows the word "comprising". Thus, the use of the term "comprising" indicates that the listed elements are required or mandatory but that other elements are optional and may or may not be present.
- The term "consisting of" means including, and limited to, whatever follows the phrase "consisting of'. Thus, the phrase "consisting of" indicates that the listed elements are required or mandatory and that no other elements may be present.
- By "therapeutically effective dose or amount" of the pharmaceutical composition of the invention is intended an amount that, when administered to the subject, brings about a positive therapeutic response in a subject suffering from cancer. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

- The term "pre-established tumorigenicity observed before transfecting the cells" typically refers to tumorigenicity observed before transfecting the cells, which is used as threshold level. The assessment of tumorigenicity can be carried out by methods known in the prior art, for instance: [*Otilia Menyhárt, Hajnalka Harami-Papp, Saraswati Sukumar, Reinhold Schäfer, Luca Magnani, Oriol de Barrios, Balázs Győrffy, Guidelines for the selection of functional assays to evaluate the hallmarks of cancer, Biochimica et Biophysica Acta (BBA) - Reviews on Cancer, Volume 1866, Issue 2, 2016, Pages 300-319, ISSN 0304-419X, https://doi.org/10.1016/j.bbcan.2016.10.002*].

- A "threshold value" or "cut-off value" can be determined experimentally, empirically, or theoretically. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative).

## Description of the figures

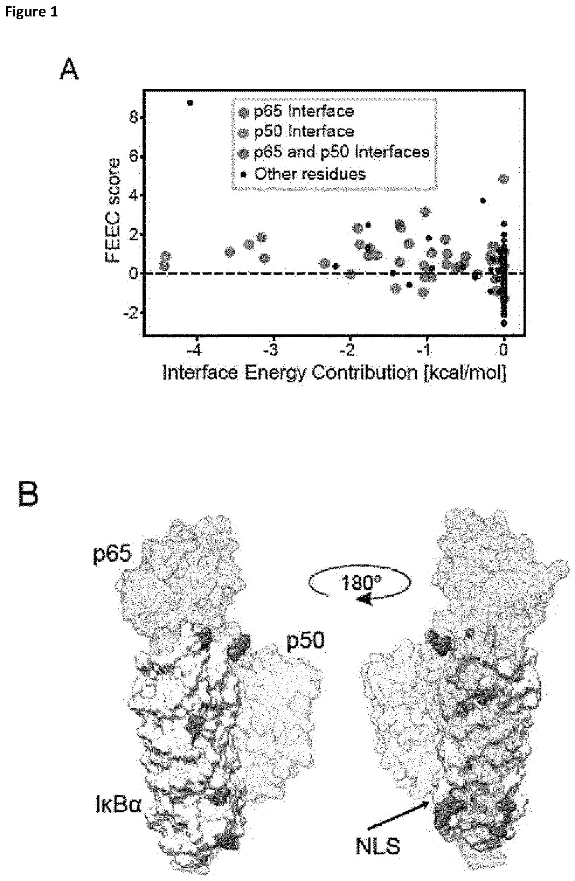

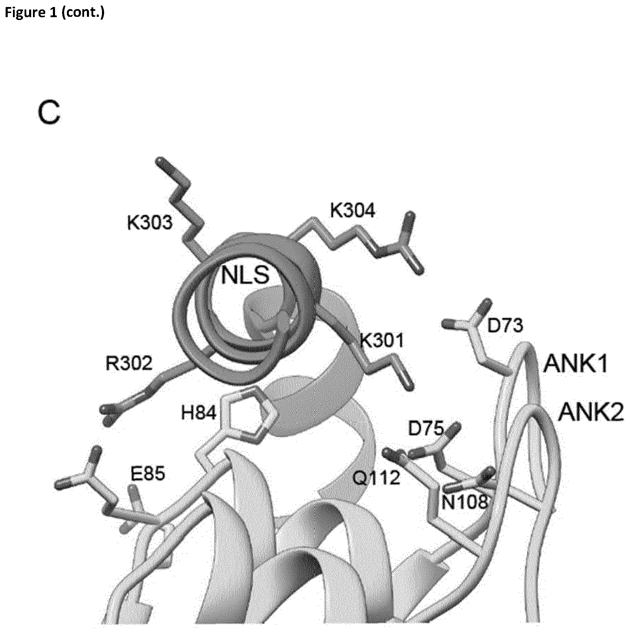

Figure 1. **A common domain of IκBα is required for p65 and histone H4 binding. (A)** Classification of IκBα and NF-κB interface residues based on the Fold-excluded Evolutionary Conservation (FEEC) score. All IκBα residue energy contributions to the interface with NF-κB are plotted against their respective FEEC scores. Interface residues between IκBα and NF-κB subunits appear in blue (p65 subunit), red (p50 subunit), or purple (p65 and p50 subunits), all remaining residues appear in black circles. **(B)** IκBα (white) and NE-κB/p65 (green) and p50 (yellow) complex. All negatively charged IκBα residues with a positive FEEC score appear as red surfaces. The NE-κB/p65 Nuclear Localization Signal (NLS) region is indicated. **(C)** Interaction of NE-κB/p65 NLS motif with IκBα ANK1 and ANK2 repeats. Polar IκBα residues interacting with the NLS are depicted in yellow, NLS motif, KRKR, is shown in green. They are numbered according to Uniprot entries Q04206 (NE-κB/p65) and P25963 (IκBα) - PDB structure 1NFI.

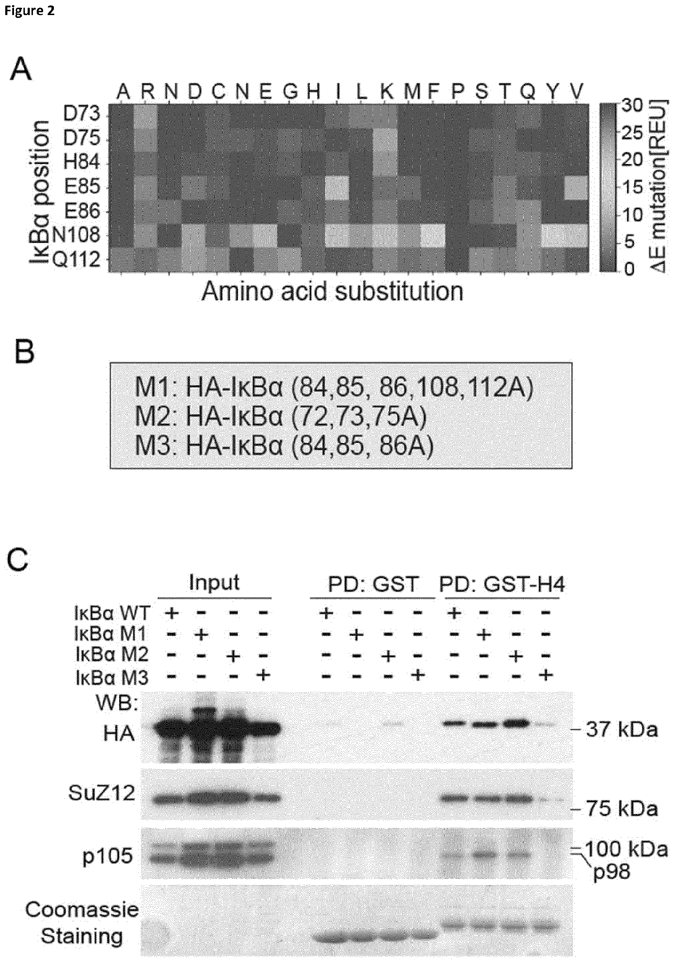

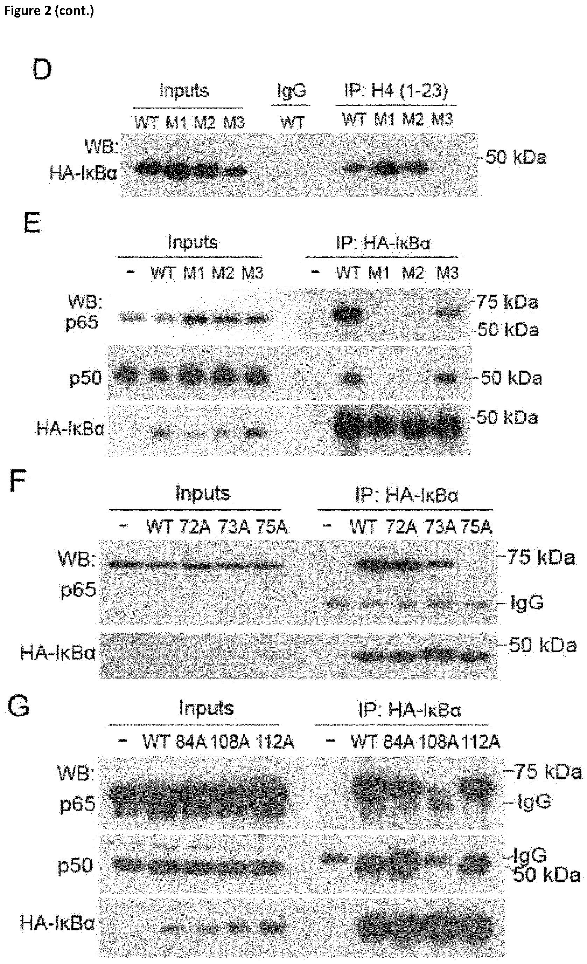

Figure 2. **Two different aminoacidic clusters in IκBα define NF-kB and PRC2 interaction. (A)** Analysis of the energy differences produced by mutating the wild type amino acid in the indicated position to any of the other 20 amino acids. **(B)** Compose mutants generated for evaluation of binding affinity with NF-κB and histone H4. **(C, D)** Precipitation assays using GST, GST-H4 as bait **(C)** or biotinylated peptides corresponding to amino acids 1-23 of H4 **(D)** and the indicated compose IκBα mutants expressed in HEK-293T cells. We determined the presence of IκBα in the precipitates by WB with anti-HA antibody. **(E)** WB analysis of the indicated NF-κB subunits in the precipitates of the indicated HA-IκBα mutants. **(F, G)** Co-IP analysis of the indicated IκBα single point mutants. Notice the total abrogation of p65 and p50 association with the single mutants D75A (**SEQ ID NO: 2**) and N108A (**SEQ ID NO: 3**). Coomassie staining in C shows the comparable amount of GST or GST-H4 in the assay.

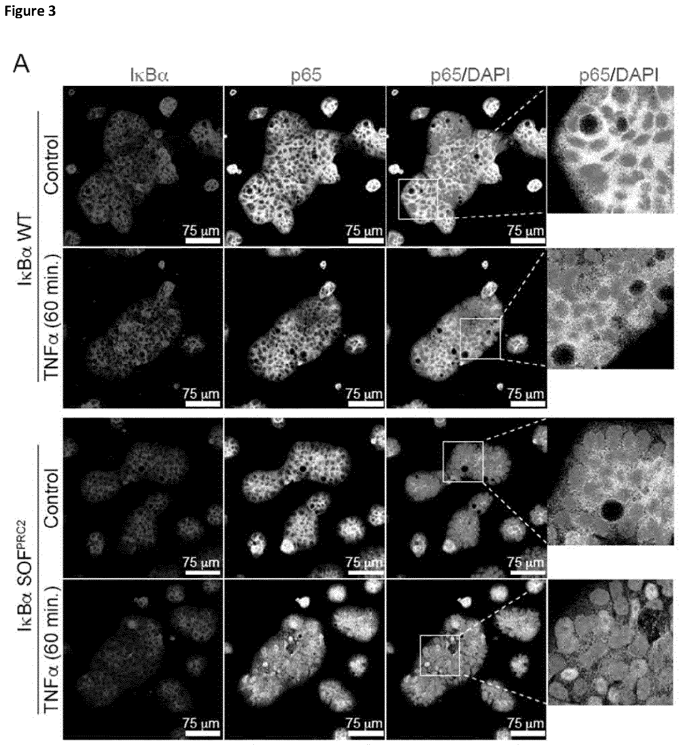

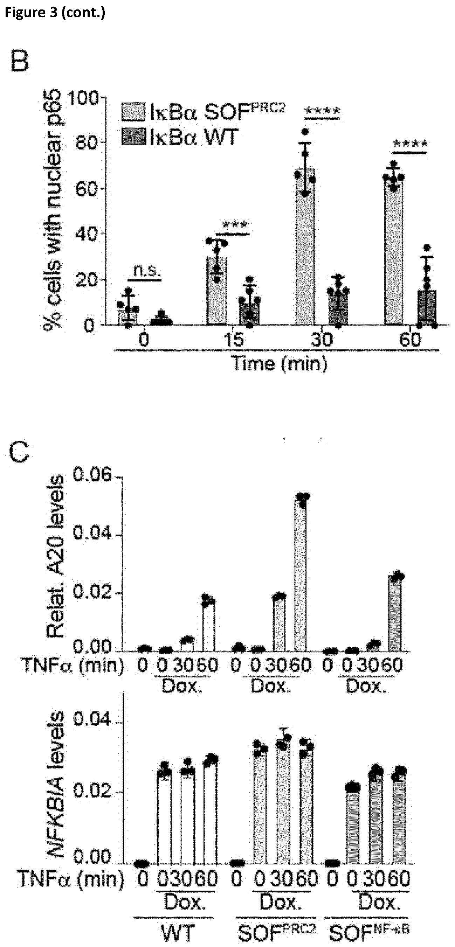

Figure 3. **IκBα mutants display separation of function (SOF) activity in cells.** (A) IF analysis of p65/NF-κB of IκBα-deficient HT29 cells reconstituted (by 16 hours doxycycline treatment) with the N108A of **SEQ ID NO: 3** or WT IκBα of **SEQ ID NO: 4** proteins and then treated with TNFα as indicated. **(B)** Quantification of the percent of cells displaying nuclear (active) p65 in cells pre-treated with doxycycline for 16 hours and then with TNFα at the indicated time points. **(C)** qPCR analysis to determine the effects of the different SOF mutants, in comparison with wildtype (WT) IκBα, in the transcriptional activation of the canonical NF-κB target gene A20. Expression levels of the different IκBα constructs are also shown in the bottom panels. Notice the higher activation of A20 in cells expressing the SOF[PRC2] mutant N108A, which is in agreement with its null capacity to negatively regulate [NF-κB].

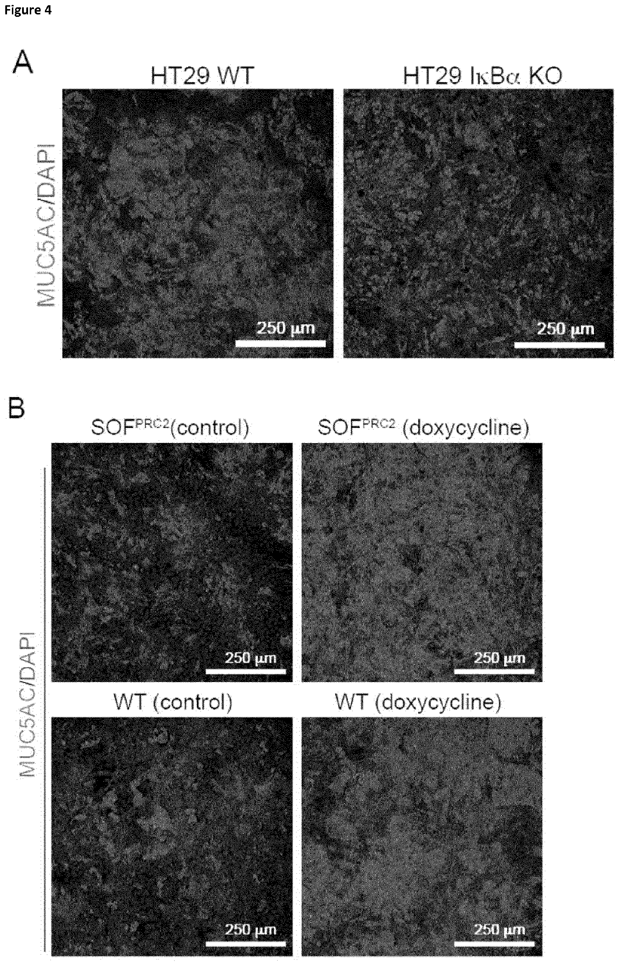

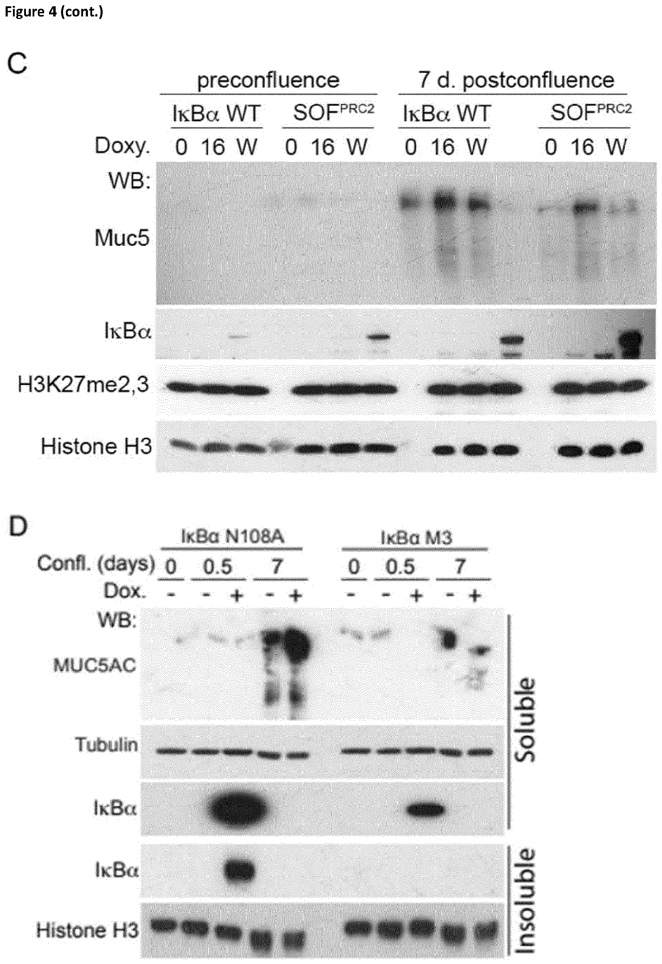

Figure 4. **IκBα SOF[PRC2] mutant N108A of SEQ ID NO: 3 is proficient in rescuing the intestinal differentiation program. (A, B)** IF analysis of the goblet cell (intestinal) differentiation marker MUC5AC in 7 days post-confluent HT29M6 WT and IκBα KO cells **(A)** or KO cells reconstituted with IκBα WT or the **SOF[PRC2]** mutant N108A (untreated or treated with doxycycline for 16 hours the day after seeding) **(B)**. **(C)** Western blot analysis of IκBα KO preconfluent or 7 days post-confluent cells reconstituted with the **SOF[PRC2]** mutant N108A and treated as indicated (0= untreated; 16= 16 hours doxycycline treatment after seeding and W= doxycycline treatment for the whole period of the experiment). **(D)** Western blot analysis of IκBα KO cells before seeding (0), preconfluent (0.5) or 7 days post-confluent cells reconstituted with the indicated IκBα SOF mutants and left untreated or treated for 16 hours with doxycycline just after seeding.

**Detailed description of the invention**

The present invention is illustrated by means of the Examples set below without the intention of limiting its scope of protection.

**Example 1. Materials and Methods**

**Example 1.1. Cell lines and reagents**

All cells were grown in Dulbecco's modified Eagle's medium (DMEM) [Invitrogen] supplemented with 10% fetal bovine serum (FBS) [Biological Industries]. Cells were grown in an incubator at 37°C and 5% $CO_2$. Cells used in these studies were HEK-293T [ATCC Ref. CRL-3216], HT29 [ATCC Ref. HTB-38D] and HCT-116 [ATCC Ref. CCL-247].

**Example 1.1. Cell transfection**

We used Polyethylenimine (PEI) [Polysciences Inc. Ref. 23996] as a carrier vector following standard methods. In brief, we diluted 4µl PEI per µg of DNA in serum-free DMEM and incubated 5 min at room temperature. Then, we added the DNA and incubated the mix 20min at RT. Finally, we incorporated the PEI/DNA solution to the cell cultures.

**Example 1.2. Pull down and peptide immunoprecipitation (IP) assays**

GST fusion proteins were incubated with lysates for 45 min in a rotary shaker at 4°C. When indicated, nuclear extracts were boiled at 98°C for 5 min in the presence of 1% SDS to disassemble pre-existing protein complexes and then neutralized in 1% Triton X-100. Precipitates were resolved in SDS-PAGE and analyzed by IB. For peptide IP, histone H4 peptides were synthesized as biotinylated N-terminal and C-terminal amides. Peptides were incubated overnight at 4°C with the indicated cell extracts and precipitated with streptavidin-Sepharose beads for 45 min.

**Example 1.3. Cell fractionation and Western Blot (WB)**

For soluble and chromatin separations, cells were lysed 1mM EDTA, 0.1mM Naorthovanadate ($Na_3VO_4$), 0.5% Triton X-100, 20mM β- glycerol-phosphate, 0.2mM PMSF, protease inhibitor cocktail, in PBS for 20 min on ice and centrifuged at 13,000 rpm. Supernatants were recovered as the soluble fraction, and the pellets were lysed in Laemmli buffer (1x SDS-PAGE buffer plus β-mercaptoethanol (BME) [Sigma, Ref. M-3148]) or in 1%SDS PBS, sonicated and treated with 1% TritonX-100. Lysates were analyzed by Western blotting using standard SDS-polyacrylamide gel electrophoresis (SDS-PAGE) techniques. In brief, protein samples were boiled in Laemmli buffer, run in polyacrylamide gels, and transferred onto polyvinylidene-difluoride (PVDF) membranes [Millipore Ref. IPVH00010]. Membranes were incubated overnight at 4°C with the appropriate primary antibodies, extensively washed and then incubated with specific secondary horseradish peroxidaselinked antibodies from Dako [Ref. P0260 and P0448]. Peroxidase activity was visualized using the enhanced chemiluminescence reagent [Biological Industries Ref. 20-500-120] and autoradiography films [GE Healthcare Ref. 28906835].

**Example 1.4. Immunofluorescence (IF) analysis**

Tissues were fixed in 4% formaldehyde overnight at room temperature and embedded in paraffin. 4µm paraffin embedded sections were first de-paraffinized in xylene. IHC was performed following standard techniques with EDTA- or citrate-based antigen retrieval and developed with the Envision+ System HRP Labelled Polymer anti-Rabbit [Dako Ref. K4003] or anti-Mouse [Dako Ref. K4001] and developed with TSA™ Plus Cyanine 3/ Fluorescein System [PerkinElmer Ref. NEL753001KT] and mounted in ProLong™ Diamond Antifade Mountant plus DAPI [Thermo Scientific Ref. P36971]. Images were taken in an SP5 upright confocal microscope (Leica).

For organoid direct immune-fluorescence, organoids were fixed with 4% paraformaldehyde, incubated with DTT buffer (100mM Tris pH9.4, 10mM DTT in $H_2O$), permeabilized with 0.5% Triton X-100 [Thermo Scientific, Ref. 28340], washed and incubated overnight with the corresponding primary antibodies. As secondary antibodies we used Alexa Fluor™ [Molecular probes, Ref.A21202 and A21206]. ProLong™ Diamond Antifade Mountant plus DAPI [Thermo Scientific Ref. P36971] was used as mounting medium. Images were taken in an SP5 upright confocal microscope (Leica).

**Example 1.5. Weighted contact number**

The weighted contact number (WCN) is a metric that quantifies the contact density for a particular atom or residue in a protein structure. It is defined as the sum of all inverse squared distances of all other particles in the system to that specific atom:

$$WCN_i = \sum_{j \neq i}^{N} \frac{1}{r_{ij}^2}$$

Here, $N$ is the number of atoms in the structure and $r_{ij}$ is the distance between atoms $i$ and $j$ The profiles are normalized by calculating their standard-score (z-score). Since we are only interested in making comparisons at the residue level, we only consider alpha carbon atoms in the protein structures.

The WCN metric, as described here, has an inverse relationship with the evolutionary sequence conservation therefore, we use the inverse of the WCN (iWCN) to make comparisons to the evolutionary information.

**Example 1.6. Sequence Conservation Score**

The sequence conservation scores were derived from the CONSURF server calculating evolutionary conservation in sequence and structure of proteins and nucleic acids. *Nucleic Acids Res.* First, the method builds phylogenetic trees from multiple sequence alignment of homologous sequences. Then, considering the stochasticity underlying the evolutionary process, conservation profiles are derived using the Empirical Bayesian Method and smoothed over a window of five residues. Finally, the scores are normalized to their corresponding standard scores; thus, the Sequence Conservation score for residue $i$ ($SC_i$) corresponds to the z-score value calculated from the distribution of CONSURF scores.

**Example 1.7. Fold-Excluded Evolutionary Conservation (FEEC) Score**

Residues conserved in a particular set of homologous proteins need to fulfill a relevant thermodynamic or kinetic role for the given biomolecular system. This role can be the maintenance of the folded structure, or it can also regulate other phenomena such as binding, catalysis, solubility, folding kinetics, etc. A three-dimensional protein structure contains the chemical contact information necessary to adopt its particular fold. The greater the number of chemical contacts a residue participates in, the more restricted it is to change its identity without compromising essential interactions that support the folded structure. Likewise, if the residue is not involved in many chemical interactions, it can vary more freely along the structurally permitted sequence landscape.

The atomic contact density, measured here as the iWCN metric, has been shown to correlate well with dynamic and sequence conservation of proteins. The later correlation is higher when the iWCN values are derived using the full biological complex structure. Since the iWCN only considers the contact information derived from a particular protein structure, residue positions with higher sequence conservations than the iWCN metric should have different or additional roles than maintaining the folded configuration (e.g., intermolecular binding). Based on this logic, we defined a Fold-Excluded Evolutionary Conservation (FEEC) score as the value of the iWCN minus the Sequence Conservation (SC) score:

$$FEEC_i = iWCN_i - SC_i$$

Based on this definition, if a residue's FEEC score is positive, it could have additional conservation constraints than those imposed by the protein's tertiary structure alone.

**Example 1.8. Classification of IκBα residues that binds NF-κB**

To validate our classification metric, we queried which IκBα residues of its interface with NF-κB were included in the set of residues with a positive FEEC score. We regarded fortyfive IκBα residues as part of its interface with NF-κB; those that had more than 20% of its Solvent Accessible Surface Area (SASA) hidden upon complexation. Thirty-six residues belong to the NE-κB/p65 subunit interface and eleven to the NF-κB/p50 subunit; two of these residues are shared between these interfaces. From the set of 213 IκBα residues in the crystallographic structure (residues indexes 70 to 282 in the IκBα sequence; Uniprot entry P25963), 121 residues have a positive FEEC score. For the p65 subunit, 83% (30/36) of the interface residues with IκBα are correctly included in this set of residues. Also, 73% (8/11) of residues in the p50 subunit interface and IκBα are within this set. Most of the interface residues not included in this set are located at the edges of the interacting surfaces (not depicted), suggesting that SASA could be an incorrect metric to classify evolutionaryrelevant interface residues because it only accounts for atoms' occlusion by the interface formation and does not consider chemical interactions. Therefore, we calculated the per-residue energy contributions of the IκBα residues to the NF-κB interfaces. We observe that all the residues left out by the classification score have less than 2 kcal/mol interface energy contributions and that all of the high contributing residues have positive FEEC scores.

**Example 1.9. Analysis of residue interface energy contributions**

Considering a protein complex based on two proteins (A and B), we assign a probability to each complex conformation from a Boltzmann distribution based on the full energy landscape:

$$P_i = \frac{e^{-E_i/KT}}{Q}$$

$E_i$ is the energy of the $i^{th}$ structure, $KT$ is the energy partition, and $Q$ is the partition function of the respective ensemble of N structures:

$$Q = \sum_i^N e^{-E_i/KT}$$

The interface binding energy $(E_i^b)$ for each complex structure is calculated as the difference between the energies of the interacting complex structure $(E_i^{complex})$ and the individual chain energies ( $E_i^A$ and $E_i^B$ ):

$$E_i^b = E_i^{complex} - \left(E_i^A + E_i^B\right)$$

All interface energies were then integrated using the complete set of probabilities to calculate the binding energy expectation value:

$$\langle E_{binding} \rangle = \sum_i^N p_i E_i^b$$

We employed the Rosetta energy function in all the modeling steps. The function is residue decomposable, and therefore it is straightforward to do the energy analyses only considering the contribution of individual residues. Accordingly, per-residue interface energy contributions are obtained as the expectation values of individual residues' binding energies. To obtain a set of interacting conformations between IκBα and NF-κB and define an interacting energy landscape, we generated 6500 minimization trajectories from the crystallographic complex (PDB code: 1NFI) using the relax protocol of the Rosetta software.

**Example 2. Results**

**Example 2.1. A common domain of IκBα is predicted to bind p65 and histone H4**

IκBα binding to histones is facilitated by limited K acetylation at the N-terminal tail of histones H2A and H4. Since bromodomain and Extraterminal (BET) family of proteins are the principal readers of KAc marks, we initially speculated that IκBα may interact with chromatin similarly to a BET protein. Pairwise sequence alignment using the EMBOSS Water Tool shows some overall similarity between the IkBa protein sequence and the regions of canonical BET protein BRD4/BRD2 involved in KAc recognition. However, the structure of the first bromodomain of BRD4 in complex with a lysine-acetylated histone H4 peptide showed that acetylated lysines are bound deep into a hydrophobic pocket formed by residues P40, F41, V45, L50, L52, Y55 and 1104 of the BRD domain (numbering according to Uniprot ID: O60885). Lysine positive charge in H4 is neutralized upon acetylation; nonetheless, polar contacts are still possible in the binding pocket and are provided by residues Y55, C94 and N98 of BRD4. This last residue makes the most significant contribution by participating with two hydrogen bond contacts with the sidechain of the acetylated lysine. Importantly, sequence similarity between the ankyrin repeats of IκBα and the BRD domain of BRD4 do not comprise residues participating in the binding of BRD4 to acetylated lysines, strongly suggesting that the mechanism by which IκBα interacts with the N-terminal portion of histone H4 is different from the binding mechanism used by bromodomains.

Based on the strict evolutionary conservation of histone tails, we then considered that residues of IκBα involved in histone H4 binding would be similarly conserved. To differentiate conserved residues of IκBα that participate in the folding of the protein from residues that could have other conserved functions (i.e. intermolecular protein-protein interactions), we derived a classification score named FEEC (Fold Excluded Evolutionary Conservation) with functional roles beyond maintaining the structural fold of the protein (for the FEEC score definition see methods). From the set of 213 IκBα residues in the crystallographic structure (aa 70 to 282 in the IκBα sequence; Uniprot entry P25963), we selected 121 residues that have positive FEEC values (**Figure 1A** and **Table 1**).

**Table 1**

| A.a. with positive FEEC | |
|---|---|
| Amino acid | FEEC Score |
| LEU70 | 1,824704653 |
| THR71 | 2,525581409 |
| GLU72 | 3,17284374 |
| ASP73 | 2,327177471 |
| GLY74 | 1,702916069 |
| ASP75 | 1,336185387 |
| SER76 | 1,230807783 |
| LEU78 | 0,598728422 |
| HIS79 | 0,797247583 |
| LEU80 | 0,771059188 |
| ALA81 | 0,903304212 |
| ILE82 | 0,17736508 |
| ILE83 | 1,114165227 |
| HIS84 | 1,858630958 |
| GLU85 | 0,535792546 |
| GLU86 | 0,476461696 |
| LEU89 | 0,271992637 |
| THR90 | 0,205569725 |
| VAL93 | 0,518912598 |
| ILE94 | 0,629637436 |
| GLN96 | 0,885767069 |
| VAL97 | 1,133099522 |
| GLY99 | 0,252762477 |
| ASP100 | 0,158808784 |
| PHE103 | 0,279646717 |
| LEU104 | 0,50377847 |
| ASN105 | 0,321115729 |

(continued)

| A.a. with positive FEEC | |
|---|---|
| Amino acid | FEEC Score |
| GLN107 | 0,171230024 |
| ASN108 | 1,138048922 |
| ASN109 | 1,032684904 |
| LEU110 | 1,060345365 |
| GLN112 | 0,645292267 |
| THR113 | 0,354764306 |
| PRO114 | 0,24780396 |
| LEU115 | 0,374878912 |
| HIS116 | 0,404649756 |
| LEU117 | 0,322244923 |
| ALA118 | 0,34684691 |
| VAL119 | 0,204050912 |
| ILE120 | 0,514411806 |
| THR121 | 1,00171294 |
| GLN123 | 0,729660754 |
| ALA127 | 0,002183166 |
| LEU130 | 0,604559319 |
| ALA133 | 0,966326028 |
| GLY134 | 1,226836884 |
| CYS135 | 0,538846225 |
| ASP136 | 0,3718363 |
| ASP141 | 0,715443834 |
| PHE142 | 0,015277468 |
| GLY144 | 0,968817011 |
| ASN145 | 0,323665058 |
| THR146 | 0,338556448 |
| HIS149 | 0,276918076 |
| ALA151 | 0,186609704 |
| LEU163 | 0,048927693 |
| THR164 | 0,122589552 |
| ASN180 | 0,726865051 |
| TYR181 | 0,895319302 |
| ASN182 | 0,175239991 |
| GLY183 | 1,055834122 |
| HIS188 | 0,370783232 |
| ALA190 | 0,178063779 |
| GLY194 | 0,038945584 |

(continued)

| A.a. with positive FEEC | |
|---|---|
| Amino acid | FEEC Score |
| LEU202 | 0,369635273 |
| GLY206 | 1,133346248 |
| ALA207 | 0,678892701 |
| ASP208 | 0,236831555 |
| VAL209 | 0,025113855 |
| ASN210 | 0,579663291 |
| GLU213 | 0,554603201 |
| PRO214 | 0,403637329 |
| CYS215 | 1,478318081 |
| ASN216 | 1,391256672 |
| GLY217 | 1,136284456 |
| ARG218 | 0,283852382 |
| THR219 | 0,220743876 |
| LEU221 | 0,476027219 |
| HIS222 | 0,490456581 |
| ALA224 | 0,455364294 |
| VAL225 | 0,386474085 |
| ASP226 | 0,906685981 |
| GLN228 | 0,37949282 |
| ASN229 | 0,240903101 |
| LEU236 | 0,479952882 |
| LEU237 | 0,419914498 |
| GLY240 | 0,9149059 |
| ALA241 | 0,404571953 |
| VAL243 | 0,743094899 |
| ASN244 | 0,916006582 |
| ARG245 | 0,323263109 |
| THR247 | 0,697931612 |
| TYR248 | 1,492286962 |
| GLN249 | 1,528452343 |
| GLY250 | 2,009908439 |
| TYR251 | 0,210412765 |
| SER252 | 0,675584032 |
| PRO253 | 0,107687365 |
| GLN255 | 0,884052646 |
| LEU256 | 0,595540376 |
| THR257 | 1,190560883 |

(continued)

| A.a. with positive FEEC | |
| --- | --- |
| Amino acid | FEEC Score |
| TRP258 | 0,401424878 |
| GLY259 | 2,351290736 |
| ARG260 | 1,73940609 |
| PRO261 | 1,315993398 |
| SER262 | 1,125626061 |
| ILE265 | 0,589234549 |
| GLN266 | 0,338927714 |
| GLN267 | 0,500083247 |
| LEU269 | 1,292318895 |
| GLN271 | 0,964356782 |
| LEU272 | 0,430829245 |
| THR273 | 2,535131546 |
| LEU274 | 2,50748884 |
| GLU275 | 1,388769794 |
| LEU277 | 0,565772862 |
| GLN278 | 0,939243145 |
| MET279 | 1,317770447 |
| LEU280 | 3,742223066 |
| PRO281 | 4,852667412 |
| GLU282 | 8,764227707 |

From this set, 38 residues are found in the IκBα and NF-κB complex interface, corresponding to 81% of the total IκBα residues in this interface. The remaining 19% of the IκBα/NF-κB interface residues showed low interaction energy values when we analyzed the crystallographic complex. This shows the potential of the FEEC score as a valuable tool to identify important functional residues.

Since the N-terminal tail of histone H4 includes a high number of positively charged residues, logical surface positions for its binding should include charge complementarity (i.e. negatively charged residues). In order to identify hot spots for interaction with H4, we considered all negatively charged surface IκBα residues within the set of the 121 residues with positive FEEC score (**Table 1**). Based on this data, the most likely position of IκBα for binding to histone H4 peptide (covering aa1-23) is coincident with a patch of the p65-NE-κB and IκBα interface (**Figure 1B**). Interestingly, this region included the Nuclear Localization Signal (NLS) of the p65-NE-κB subunit (residues 301 to 304 (KKRK)). A sequence alignment between this region and the H4 N-terminal tail revealed an alpha-helical motif that is conserved between the two proteins. This NLS domain of p65-NE-κB makes contact with many IκBα charged and polar residues, including D73, D75, H84, E85, E86, N108 and Q112 that belong to the ANK1 and ANK2 subdomains (**Figure 1C**). Different secondary structure prediction servers also showed a comparable helical character for the region between K16 and K20 of histone H4. We propose that IκBα residues interacting with p65-NE-κB could also mediate the interaction with histone H4.

**Example 2.2. Two different aminoacidic clusters in IκBα define NF-kB and PRC2 interaction.**

Consistent with their functional relevance, IκBα residues candidate to mediate NF-κB and histone H4 binding were evolutionary conserved from nematodes to humans. To directly investigate whether D73, D75, H84, E85, E86, N108 and Q112 were essentially contributing to mediate NF-κB or histone H4 binding, we designed IκBα variants carrying specific combinations of mutations for their use in co-precipitation (Co-IP) and pull-down (PD) assays. With this aim, we first carried out a mutational analysis, over the structure of IκBα, to determine the effect of changing these positions to non-synonymous residues. The protocol includes the minimization of the wild type structure and the generation of selected mutants by changing the identity of the target residue. This is followed by repacking and backbone minimization of the target and neighbouring residues until a radius of 8 angstroms. The energy differences between mutating to the wild type amino acid and to any of the other 20 amino acids are reported (**Figure 2A**). According to the mutation matrix, all positions will accept an alanine mutation without a big change in the energy of the system whereas proline mutations are forbidden except in position N108. Based on this data, we generated different compose mutants carrying alanine substitutions (**Figure 2B**).

By PD assay, we found that mutation of N72, D73 and D75 to A did not affect IκBα binding to histone H4, which was almost abolished by H84, E85, E86 to A mutations (**Figure 2C**). Identical results were obtained by co-IP with peptides corresponding to the N terminal tail of histone H4 (Aa 1-23) (**Figure 2D**). We then performed Co-IP assay from transfected HEK-293Tcells to test the binding capacity of IκBα mutants with endogenous NF-κB subunits. We found reduced association of H84, E86A mutant to p65/NE-κB compared with WT IκBα of **SEQ ID NO: 4**, whereas mutants N72, D73, D75 to A and H84, E85, E86, N108, Q112 to A displayed an absolute lack of p65 and p50 NF-κB binding (**Figure 2E**). We then generated single point mutants for all candidate residues to further refine our data.

By PD and Co-IP experiments we uncover residues D75 and N108 as essential drivers of IκBα association to NF-κB (**Figures 2F and 2G**), whereas single mutation of H84, E85 or E86 to A did not affect histone H4 binding compared with the compose H84, E85, E86 to A mutant (**not shown**). In the same experiments, we confirmed increased association of the PRC2 element SuZ12 in the presence of IκBα that was similarly observed for different histone binding proficient IκBα mutants (**not shown**). From now on, we will name IκBα SOF^PRC2 those mutants displaying specific and exclusive histone and PRC2 binding D75A (**SEQ ID NO: 2**) or N108A (**SEQ ID NO: 3**) and SOF^NF-κB the IκBα mutant that fails to bind histones and PRC2 but associates with p65 and p50 (H84, E85, E86 to A) (**SEQ ID NO: 1**). Together these results support the concept that p65-NE-κB and histone H4 competes for binding to the same IκBα region, but specific mutations differentially impact on p65-NE-κB and histone H4 association.

**Example 2.3. IκBαmutants display separation of function (SOF) activity in cells**

We then experimentally studied the biochemical properties and functional activity of candidate SOF^PRC2 (**SEQ ID NO: 2** or **SEQ ID NO: 3**) and SOF^NF-κB (**SEQ ID NO: 1**) IκBα mutants in cells. Since negative regulation of NF-κB is the primary function assigned to IκBα, we tested whether IκBα SOF^PRC2 (**SEQ ID NO: 2** or **SEQ ID NO: 3**) was capable to prevent TNFα-induced nuclear translocation of p65/RelA. We generated doxycycline-inducible WT (**SEQ ID NO: 4**) and IκBα SOF^PRC2 (**SEQ ID NO: 2** or **SEQ ID NO: 3**) lines in IκBα-deficient HT29 colorectal cancer cells. We induced IκBα expression by 16 hours incubation with doxycycline in the culture medium and then we left cells untreated or treated with TNFα for 30 and 60 minutes. By IF analysis, we found that ectopic expression of IκBα WT of **SEQ ID NO: 4** in IκBα-deficient cells totally abrogated the nuclear translocation of p65 after TNFα treatment (**Figure 3A**, upper panels). In contrast, ectopic expression of the IκBα SOF^PRC2 mutant N108A (**SEQ ID NO: 3**) did not prevent p65 translocation (**Figure 3A**, lower panels), consistent with NF-κB signalling being blind to IκBα SOF^PRC2 mutants. We quantified the percent of cells displaying nuclear p65 at different time-points after TNFα treatment (**Figure 3B**). Then, we performed comparable experiments to measure NF-κBdependent transcription in IκBα WT, SOF^PRC2 and SOF^NF-κB-reconstituted cells by qPCR analysis of the canonical NF-κB target gene A20. Our results indicated that IκBα WT and IκBα SOF^NF-κB both delayed transcriptional induction of A20 gene compared with the NF-κB-defective SOF^PRC2 mutant N108A (**Figure 3C**).

**Example 2.4. IκBα SOF^PRC2 mutant N108A (SEQ ID NO: 3) is proficient in rescuing stem cell programs and intestinal differentiation**

We previously demonstrated that intestinal cells lacking IκBα display defective adult intestinal stem cell gene signatures and fail to differentiate towards the intestinal lineages. We now confirmed that IκBα KO HT29M6 cells show defective goblet cell differentiation as indicated by the reduced levels of the differentiation marker MUC5AC (**Figure 4A**). We have now reconstituted HT29 IκBα KO cells with doxycycline-inducible WT (**SEQ ID NO: 4**), SOF^PRC2 (**SEQ ID NO: 2** or **SEQ ID NO: 3**) and SOF^NF-κB (**SEQ ID NO: 1**) IκBα mutants and tested their differential capacity for goblet cell differentiation. We initially tested two different IκBα induction protocols, 16 hours doxycycline treatment at pre-confluence and then maintained the cultures for 7 days of post-confluence (in these conditions IκBα WT HT29 cells differentiate and express high levels of MUC2 and MUC5), or doxycycline treatment for the whole period of the experiment. We found that 16 hours of induction with the IκBα SOF^PRC2 mutant N108A (**SEQ ID NO: 3**) was sufficient to promote HT29 cell differentiation, which was even higher than the effect of the WT construct of **SEQ ID NO: 4** (**Figure 4B and 4C**). We performed comparable differentiation experiments with IκBα SOF^PRC2 and SOF^NF-κB reconstituted HT29M6 cells. As shown in **Figure 4D,** expression levels of the differentiation marker MUC5AC were highly reduced in cells reconstituted with SOF$^{NF-\kappa B}$ compared with SOF$^{PRC2}$. These results indicate that defective differentiation in the I$\kappa$B$\alpha$ KO cells is NF-$\kappa$B independent but I$\kappa$B$\alpha$/PRC2 dependent.

**Example 2.5. Expression of I$\kappa$B$\alpha$ wild type, SOF$^{PRC2}$ or SOF$^{NF-\kappa B}$ I$\kappa$B$\alpha$ mutants into highly aggressive I$\kappa$B$\alpha$-deleted GBM cells and tumorigenic activity testing of all edited lines *in vitro* and *in vivo***

We will express I$\kappa$B$\alpha$ wild type, SOF$^{PRC2}$ or SOF$^{NF-\kappa B}$ I$\kappa$B$\alpha$ mutants into highly aggressive I$\kappa$B$\alpha$-deleted GBM cells and will test tumorigenic activity of all edited lines *in vitro* and *in vivo*. We will perform RNA-sequencing of I$\kappa$B$\alpha$-reconstituted cells and parental I$\kappa$B$\alpha$-deleted cells. Expected results: Analysis of differentially expressed genes (DEG) identifies genes 1 to 120 as upregulated in I$\kappa$B$\alpha$-deleted compared with I$\kappa$B$\alpha$ wild type reconstituted cells. Genes 1 to 60 are repressed in SOF$^{PRC2}$ (polycomb targets) cells and genes 61 to 120 are repressed in SOF$^{NF-\kappa B}$ cells (NF-$\kappa$B targets). SOF$^{PRC2}$ (and probably I$\kappa$B$\alpha$ wild type) preclude tumorigenic GBM activity thus indicating a tumour-suppressor activity of genes 1-60. Gene Set Enrichment Analysis demonstrates that genes 1-60 are primarily involved in negative regulation of PI3K and Wnt pathways. From this data we can stablish that: i) genes 1-60 are candidate biomarkers to identify tumours with defective I$\kappa$B$\alpha$-PRC2-related activity; ii) patients with tumours expressing 1-60 biomarkers are candidates to be treated with approved therapeutic compounds targeting PRC2 and iii) PI3K and Wnt inhibitors can be proposed as alternative treatment for these same patients. A comparable approach can be used in tumours that are rescued by SOF$^{NF-\kappa B}$,

**Claims**

1.  I$\kappa$B$\alpha$ mutant consisting of SEQ ID NO: 1, SEQ ID NO: 2 or SEQ ID NO: 3.

2.  Genetic vector encoding the I$\kappa$B$\alpha$ mutants of claim 1.

3.  *In vitro* method for deciding or recommending a treatment to a patient suffering cancer, wherein the cancer is **characterized by** the inactivation of I$\kappa$B$\alpha$ protein, which comprises:

    a. Transfecting tumor cells obtained from the patient with a genetic vector which encodes I$\kappa$B$\alpha$ mutant consisting of SEQ ID NO: 1, SEQ ID NO: 2 or SEQ ID NO: 3;
    b. Wherein the identification of a reduction or elimination of tumorigenicity once the tumor cells have been transfected with a genetic vector encoding I$\kappa$B$\alpha$ mutant consisting of SEQ ID NO: 1, as compared with the tumorigenicity observed before transfecting the cells, is an indication that the tumor has been originated via activation of NF-$\kappa$B, and a treatment directed to the inhibition of NF-$\kappa$B pathway would be recommended; or
    c. Wherein the identification of a reduction or elimination of tumorigenicity once the tumor cells have been transfected with a genetic vector encoding I$\kappa$B$\alpha$ mutant consisting of SEQ ID NO: 2 or SEQ ID NO: 3, as compared with the tumorigenicity observed before transfecting the cells, is an indication that the tumor has been originated via PRC2, and a treatment directed to the inhibition of PRC2 pathway would be recommended.

4.  *In vitro* method for the identification of therapeutic targets or biomarkers for the treatment or diagnosis of a cancer type **characterized by** the inactivation of I$\kappa$B$\alpha$ protein, which comprises:

    a. Performing a transcriptomic or proteomic study of reconstituted cells transfected with a genetic vector encoding I$\kappa$B$\alpha$ mutant consisting of SEQ ID NO: 1, SEQ ID NO: 2 or SEQ ID NO: 3;
    b. Performing a transcriptomic or proteomic study of the cells before being transfected;
    c. Wherein the identification of a deviation in the level of expression of a particular gene or protein, is an indication that the protein or gene can be used as a therapeutic target or biomarker for the treatment or diagnosis of a cancer type **characterized by** the inactivation of I$\kappa$B$\alpha$ protein.

5.  In vitro method, according to any of the claims 3 or 4, wherein the cancer type **characterized by** the inactivation of I$\kappa$B$\alpha$ protein is selected from glioblastoma, low grade glioma, non-small cell lung carcinoma, gray zone lymphoma or Hodgkin lymphoma.

6.  NF-$\kappa$B inhibitors for use in the treatment of patients suffering from a cancer type **characterized by** the inactivation of I$\kappa$B$\alpha$ protein, which comprises assessing whether the cancer has been actually originated via activation of NF-$\kappa$B by transfecting patient cells with a genetic vector encoding the I$\kappa$B$\alpha$ mutant consisting of SEQ ID NO: 1.

7. PRC2 inhibitors for use in the treatment of patients suffering from a cancer type **characterized by** the inactivation of IκBα protein, which comprises assessing whether the cancer has been actually originated via activation of PRC2 by transfecting patient cells with a genetic vector encoding a IκBα mutant consisting of SEQ ID NO: 2 or SEQ ID NO: 3.

**Figure 1**

**Figure 1 (cont.)**

**Figure 2**

A

B

M1: HA-IκBα (84,85, 86,108,112A)
M2: HA-IκBα (72,73,75A)
M3: HA-IκBα (84,85, 86A)

C

Figure 2 (cont.)

**Figure 3**

**Figure 3 (cont.)**

Figure 4

**Figure 4 (cont.)**

**EUROPEAN SEARCH REPORT**

Application Number

EP 22 38 2186

## DOCUMENTS CONSIDERED TO BE RELEVANT

| Category | Citation of document with indication, where appropriate, of relevant passages | Relevant to claim |
|---|---|---|
| X | HUXFORD T ET AL: "Solvent Exposed Non-contacting Amino Acids Play a Critical Role in NF-@kB/I@kB@a Complex Formation", JOURNAL OF MOLECULAR BIOLOGY, ACADEMIC PRESS, UNITED KINGDOM, vol. 324, no. 4, 6 December 2002 (2002-12-06), pages 587-597, XP004449966, ISSN: 0022-2836, DOI: 10.1016/S0022-2836(02)01149-X | 1-4,7 |
| Y | * PG 587, 589, 590, 594; | 5 |
| A | table 1 * | 6 |
| | ----- | |
| A | MULERO MARÍA CARMEN ET AL: "Chromatin-Bound I[kappa]B[alpha] Regulates a Subset of Polycomb Target Genes in Differentiation and Cancer", CANCER CELL, vol. 24, no. 2, 12 August 2013 (2013-08-12), pages 151-166, XP55947527, US ISSN: 1535-6108, DOI: 10.1016/j.ccr.2013.06.003 Retrieved from the Internet: URL:https://www.sciencedirect.com/science/article/pii/S1535610813002791/pdfft?md5=d29db7c7b14ddbdc0ef9cf19c2a32cbf&pid=1-s2.0-S1535610813002791-main.pdf> * page 155 - page 157 * | 1-7 |
| | ----- | |
| | -/-- | |

**CLASSIFICATION OF THE APPLICATION (IPC)**

INV.
G01N33/50

ADD.
C12Q1/6886
G01N33/574

**TECHNICAL FIELDS SEARCHED (IPC)**

G01N
C12Q
A61P

The present search report has been drawn up for all claims

| Place of search | Date of completion of the search | Examiner |
|---|---|---|
| The Hague | 3 August 2022 | Kosten, Jonas |

EPO FORM 1503 03.82 (P04C01)

page 1 of 3

**EP 4 239 334 A1**

Europäisches Patentamt

European Patent Office

Office européen des brevets

## EUROPEAN SEARCH REPORT

Application Number

EP 22 38 2186

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ESPINOSA LLUÍS ET AL: "NF-[kappa]B-Dependent and -Independent (Moonlighting) I[kappa]B[alpha] Functions in Differentiation and Cancer", BIOMEDICINES, vol. 9, no. 9, 21 September 2021 (2021-09-21), page 1278, XP055946978, DOI: 10.3390/biomedicines9091278 Retrieved from the Internet: URL:https://www.ncbi.nlm.nih.gov/pmc/artic les/PMC8468488/pdf/biomedicines-09-01278.p df> | 5 | |
| A | * page 6; table 1 * | 1-4,6,7 | |
| A | UEMATSU ATSUSHI ET AL: "DANFIN functions as an inhibitor of transcription factor NF-[kappa]B and potentiates the antitumor effect of bortezomib in multiple myeloma", BIOCHEMICAL AND BIOPHYSICAL RESEARCH COMMUNICATIONS, ELSEVIER, AMSTERDAM NL, vol. 495, no. 3, 25 December 2017 (2017-12-25), pages 2289-2295, XP085327777, ISSN: 0006-291X, DOI: 10.1016/J.BBRC.2017.12.142 * figure 1 * | 1-7 | TECHNICAL FIELDS SEARCHED (IPC) |

-/--

The present search report has been drawn up for all claims

| Place of search | Date of completion of the search | Examiner |
|---|---|---|
| The Hague | 3 August 2022 | Kosten, Jonas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

26

Europäisches
Patentamt

European
Patent Office

Office européen
des brevets

# EUROPEAN SEARCH REPORT

**Application Number**

EP 22 38 2186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KRISHNAN JAYALAKSHMI ET AL: "Drugs targeting toll-like receptors", ARCHIVES OF PHARMACAL RESEARCH, NATL. FISHERIES UNIVERSITY , PUSAN, KR, vol. 32, no. 11, 20 November 2009 (2009-11-20), XP037138291, ISSN: 0253-6269, DOI: 10.1007/S12272-009-2100-6 [retrieved on 2009-11-20] * page 1494 * | 1-7 | |
| A | THOMAS-JARDIN SHAYNA E ET AL: "NF-[kappa]B signaling promotes castration-resistant prostate cancer initiation and progression", PHARMACOLOGY & THERAPEUTICS, ELSEVIER, GB, vol. 211, 19 March 2020 (2020-03-19), XP086157851, ISSN: 0163-7258, DOI: 10.1016/J.PHARMTHERA.2020.107538 [retrieved on 2020-03-19] * page 4 * | 1-7 | |
| | | | **TECHNICAL FIELDS SEARCHED (IPC)** |

The present search report has been drawn up for all claims

| Place of search | Date of completion of the search | Examiner |
|---|---|---|
| The Hague | 3 August 2022 | Kosten, Jonas |

EPO FORM 1503 03.82 (P04C01)

page 3 of 3

Europäisches
Patentamt

European
Patent Office

Office européen
des brevets

**Application Number**

EP 22 38 2186

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 22 38 2186

The Search Division considers that the present European patent application does not comply with the requirements of unity of invention and relates to several inventions or groups of inventions, namely:

```
1. claims: 1-7

   Methods and products related to I?B? mutants.

1.1. claims: 6(completely); 1-5(partially)

   The claims as so far as they relate to SEQ1 (HEE84-86AAA).

1.2. claims: 1-5, 7(all partially)

   The claims as so far as they relate to SEQ2 (D75A).

1.3. claims: 1-5, 7(all partially)

   The claims as so far as they relate to SEQ3 (N108A).
                       ---

Please note that all inventions mentioned under item 1, although not
necessarily linked by a common inventive concept, could be searched
without effort justifying an additional fee.
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **EMMERICH F ; MEISER M ; HUMMEL M et al.** Overexpression of I kappa B alpha without inhibition of NF-kappaB activity and mutations in the I kappa B alpha gene in Reed-Sternberg cells. *Blood,* 01 November 1999, vol. 94 (9), 3129-34
- **CABANNES E ; KHAN G ; AILLET F ; JARRETT RF ; HAY RT.** Mutations in the IkBa gene in Hodgkin's disease suggest a tumour suppressor role for IkappaBalpha. *Oncogene,* 20 May 1999, vol. 18 (20), 3063-70
- **JUNGNICKEL B ; STARATSCHEK-JOX A ; BRÄUNINGER A et al.** Clonal deleterious mutations in the IkappaBalpha gene in the malignant cells in Hodgkin's lymphoma. *J Exp Med.,* 17 January 2000, vol. 191 (2), 395-402
- **ZHAO Z ; ZHONG X ; WU T et al.** Identification of a NFKBIA polymorphism associated with lower NFKBIA protein levels and poor survival outcomes in patients with glioblastoma multiforme. *Int J Mol Med.,* November 2014, vol. 34 (5), 1233-40
- **BREDEL M ; SCHOLTENS DM ; YADAV AK et al.** NFKBIA deletion in glioblastomas. *N Engl J Med.,* February 2011, vol. 364 (7), 627-37
- **HE Y ; ZHANG H ; YIN J et al.** IkappaBalpha gene promoter polymorphisms are associated with hepatocarcinogenesis in patients infected with hepatitis B virus genotype C. *Carcinogenesis,* November 2009, vol. 30 (11), 1916-22
- **PARKER KM ; MA MH ; MANYAK S et al.** Identification of polymorphisms of the IkappaBalpha gene associated with an increased risk of multiple myeloma. *Cancer Genet Cytogenet.,* August 2002, vol. 137 (1), 43-8
- **MULERO MC ; FERRES-MARCO D ; ISLAM A et al.** Chromatin-bound IκBα regulates a subset of polycomb target genes in differentiation and cancer. *Cancer Cell,* August 2013, vol. 24 (2), 151-66
- **YU H ; LIN L ; ZHANG Z ; ZHANG H ; HU H.** Targeting NF-κB pathway for the therapy of diseases: mechanism and clinical study. *Signal Transduct Target Ther.,* 21 September 2020, vol. 5 (1), 209
- **DURAND JK ; BALDWIN AS.** Targeting IKK and NF-κB for Therapy. *Adv Protein Chem Struct Biol.,* 2017, vol. 2017 (107), 77-115
- **HE C ; WANG M, et al.** Targeting epigenetic regulators for cancer therapy: mechanisms and advances in clinical trials. *Signal Transduct Target Ther. 2019,* 2019, vol. 4, 62
- **DAWSON MA ; KOUZARIDES T.** Cancer epigenetics: from mechanism to therapy. *Cell,* 06 July 2012, vol. 150 (1), 12-27
- **PIUNTI A ; HASHIZUME R ; MORGAN MA et al.** Therapeutic targeting of polycomb and BET bromodomain proteins in diffuse intrinsic pontine gliomas. *Nat Med.,* April 2017, vol. 23 (4), 493-500